# EUROPEAN PATENT SPECIFICATION

(11) **EP 3 335 729 B1**
(45) Date of publication and mention of the grant of the patent: **28.06.2023**
(21) Application number: 16835520.4
(22) Date of filing: 09.08.2016
(51) Int. Cl.: A61K 39/395, A61K 47/18, A61K 47/02, A61K 47/26, A61K 47/10, A61K 9/08

(54) **ETANERCEPT COMPOSITION HAVING IMPROVED STABILITY**
ETANERCEPTZUSAMMENSETZUNG MIT VERBESSERTER STABILITÄT
COMPOSITION À STABILITÉ AMÉLIORÉE D'ÉTANERCEPT

(30) Priority: 13.08.2015 MX 2015010517
(43) Date of publication of application: 20.06.2018
(73) Proprietor: Landsteiner Scientific S.A. de C.V., 01900 Distrito Federal (MX)
(72) Inventor: KURI BREÑA ROMERO DE TERREROS, Francisco, Ciudad de México 01900 (MX)
(74) Representative: Izquierdo Blanco, Maria Alicia
(86) International application number: PCT/MX2016/000082
(87) International publication number: WO 2017/026881

(56) References cited:
- WO-A1-2012/165917
- WO-A2-03/072060
- US-A1- 2013 101 583
- US-A1- 2013 101 640
- US-A1- 2015 125 532
- GOKARN Y R ET AL: "Excipients for protein drugs", 1 January 2006 (2006-01-01), EXCIPIENT DEVELOPMENT FOR PHARMACEUTICAL, BIOTECHNOLOGY, AND DRUG DELIVERY SYS,, PAGE(S) 291 - 331, XP009179656, * the whole document * * page 310 - page 310 *
- ANDREAS ZIMMER: "Galenische Formulierung rekombinanter Wirkstoffe: Problem Arzneistoffstabilit?", PHARMAZIE IN UNSERER ZEIT, vol. 32, no. 5, 1 September 2003 (2003-09-01), pages 384-389, XP055109543, ISSN: 0048-3664, DOI: 10.1002/pauz.200300037

## Description

### Invention Field

This invention refers to an aqueous formulation of Etanercept for the production of injectable solutions, whose improved stability reduces the chemical and physical denaturation of Etanercept during storage.

### Background Information

Etanercept is a biological inflammation modulator that functions as a competitive TNF-alpha inhibitor that binds to the TNF-alpha receptor cell surface to inhibit immune responses mediated by TNF-alpha. Consequently, Etanercept is used to treat rheumatoid arthritis, psoriasis and ankylosing spondylitis. As a protein drug, Etanercept has an average short life and is chemically and physically denatured easily, since it is susceptible to changes in climate temperature, shear stress, vibration, freezing/thawing, UV exposure, pH changes, organic solvents and microbial contamination. Chemical denaturation includes dissociation of the dimers that comprise the Etanercept molecule, oxidation, deamination, isomerization and polymerization and by the different antibody attributes -salt, pH and temperature-. Also, chemical denaturation includes the loss of tertiary structure, covalent/non-covalent aggregation and adhesion of monomers. The physical and chemical denaturation of Etanercept causes a loss of its physiological activity, since denaturation is an irreversible process, which causes the reduction of its therapeutic efficacy.

Consequently, several studies have been carried out on Etanercept formulations containing physiologically effective amounts without aggregates. Suitable stabilizers are required In order to prepare stable formulations. Until now, the use of combinations of stabilizers causes competition between them and the adverse effects may cause undesired effects.

There are a few studies on stable liquid Etanercept formulations; the only stabilization procedure is through the use of amino acids such as I-arginine, in combination with sucrose and a phosphate buffer.

Patent MX263,192 protects an aqueous pharmaceutical composition suitable for long-term storage containing a TNF receptor polypeptide fused to an Fc domain of an immunoglobulin (TNFR:Fc). This patent also includes a method for formulating said pharmaceutical composition by combining TNFR.Fc isolated from L-arginine, in addition to the steps for combining a pH regulator, a tonicity modifier and an excipient with the composition. In another embodiment, the patent describes a device or container which contains the pharmaceutical composition. In the application MX/a/2014/000093 directed at compositions containing arginine-free polypeptides, and at methods for treating disorders associated with inflammation or immune response; in particular, the polypeptide is Etanercept. Application MX/a/2013/014027 refers to a stable liquid formulation of Etanercept (recombinant sTNFR:Fc p75 fusion protein) and more particularly to a liquid formulation comprising one or more stabilizers selected from the group, which consists of methionine, histidine and their pharmaceutically acceptable salts in an amount sufficient to reduce the formation of Etanercept by-products during storage.

This liquid formulation effectively reduces Etanercept by-product production and maintains its stable pharmaceutical efficacy during long-term storage. Therefore, a reconstitution procedure is not required before its administration and the sterile formulation can be administered to patients to ensure patient safety.

The WO2014177548 (A1) 2014-11-06 publication provides a stable liquid formulation allowing for the storage of TNFRiFc polypeptides. The stable aqueous compositions are free of arginine and cysteine, and are also very stable at high temperatures. These compositions comprise a salt amount similar to the physiological body salt concentration. The aqueous composition comprises: an isolated polypeptide which is an extracellular binding portion of a human p75 tumor necrosis factor receptor fused to the Fc region of a human IgGI; salt in a concentration of 80 to 130 mm; and an excipient selected from the trehalose and sucrose group and their combinations, characterized in that neither arginine nor cysteine are present in the composition.

Patent EP 2768535 (A4) 2015-03-11; "Etanercept Formulations Stabilized with Xylitol", provides stable liquid Etanercept formulations which support long-term storage. The stabilized aqueous pharmaceutical composition which comprises Etanercept and a stabilizer for inhibiting the instability, aggregation, misfolding and/or fragmentation of Etanercept, wherein the stabilizer comprises xylitol or a combination of xylitol and meglumine.

In GOKARN Y R ET AL: "Excipients for protein drugs", 1 January 2006 (2006-01-01), "Excipient development for pharmaceutical, biotechnology, and drug delivery sys" page(s) 291-331 XP009179656, discloses lyophilisates and a liquid solution of the claimed active etanercept (Enbrel Lyo and Enbrel sol). This document fails to disclose a solution comprising the claimed compounds (no mannitol).

In ANDREAS ZIMMER: "Galenische Formulierung rekombinanter Wirkstoffe: Problem Arzneistoffstabilit?", pharmazie in unserer zeit, vol. 32, no. 5, 1 September 2003 (2003-09-01), pages 384-389, XP055109543, ISSN: 0048-3664, 001: 10.1002/pauz.200300037, discloses various compositions comprising proteins such as Enbrel Lyo comprising mannitol and saccharose but fails to disclose all other claimed compounds such as arginine. This document discloses only the Enbrel Lyo.

In WO 03/072060 (A2) (2003-09-04) disclose mannitol and sucrose as tonicity modifier but this document is silent about a combined use of sucrose and mannitol in a single composition. This document further discloses that commercially available TNFR: Fc is known as etanercept. This does not teach or suggest the joint use of mannitol or sucrose. The formulations described in this document, exemplify the content of excipients in the composition, thus when mannitol is used the content is 5%, and when sucrose is used the amount is 10%, consequently only one of these sugars is present in the composition, the use of mannitol and sucrose together in the disclosed and claimed compositions is not taught or suggested. In this document it is clear to use one excipient selected from those indicated, where it is possible to select sucrose or mannitol, by no means both saccharides.

In EP2768533 (A1) 2014-08-27 EP2768533 (A4) 2015-03-11; "Etanercept Formulations Stabilized with Combinations of Sugars and Polyols", where the Etanercept formulations are stabilized and are preferably free of arginine, with which a long-term stabilization is achieved.

The WO2012143418 (A1) 2012-10-26; "Stable Liquid Formulations of TNFR.Fc Fusion Proteins" publication refers to stable pharmaceutical liquid formulations of the TNFR:Fc fusion protein comprising different buffer systems and stabilizers, in particular physical TNFR:Fc stability is significantly improved by the use of a buffer system of citrate and lysine and/or proline as a stabilizer.

Patent US7507745 (B2) 2009-03-24, "Pharmaceutical Compositions Based on Fluorocarboxylic Acid Esters and Receptors of Soluble TNF Fusion Proteines" describes a pharmaceutical composition which may comprise TNFR:Fc; the citrate buffer can be any suitable citrate buffer; an amino acid selected from the lysine and proline group and their pharmaceutically acceptable salts, such as hydrochlorides; in addition, the pharmaceutical composition may comprise at least one tonicity modifier, preferably, at least one tonicity modifier is selected from the sodium chloride, cysteine, histidine, glycine, potassium chloride, sucrose, glucose and mannitol group, with preference for sodium chloride and/or sucrose. The composition may also comprise at least one excipient, wherein at least one excipient may be selected from the group consisting of lactose, glycerol, xylitol, sorbitol, mannitol, maltose, inositol, trehalose, glucose, bovine serum albumin (BSA), dextran, polyvinyl acetate (PVA), hydroxypropyl methylcellulose (HPMC), polyethylenimine (PEI), gelatine, polyvinylpyrrolidone (PVP), hydroxyethylcellulose (HEC), polyethylene glycol (PEG), ethylene glycol, glycerol, dimethylsulfoxide (DMSO), dimethylformamide (DMF), L-serine, sodium glutamate, alanine, sorbitan monolaurate, polyoxyethylene glycine, sarcosine, gamma-aminobutyric acid (GABA) (Tween-20), polyoxyethylene sorbitan-monooleate (Tween-80), sodium dodecyl sulphate (SDS), polysorbate , polyoxyethylene copolymer, potassium phosphate, sodium acetate, ammonium sulphate, magnesium sulphate, sodium sulphate, trimethylamine N-oxide, betaine, zinc ions, copper ions, calcium ions, manganese ions, magnesium ions, CHAPS, m sucrose onolaurate and 2-O-beta-mannoglycerate.

Currently, for example, the TNFR:Fc protein, Etanercept, is marketed under the Enbrel^{®} trade name which has the following composition and which is protected by the MX263192 patent:
Composition of Etanercept (Enbrel^{®})

| | |
|---|---|
| Etanercept | 50 Mg/ml; 0.3 mm |
| Etanercept | 10 Mg/ml; 29 mm |
| Sodium Chloride | 5.8 Mg/ml; 100 mm |
| L-Arginine Hydrochloride | 5.3 Mg/ml; 25 mm |
| Sodium Chloride | 2.6 Mg/ml; 19 mm |
| Disodium Phosphate | 0.9 Mg/ml; 6 mm |
| Purified Water pH 6.3 ± 0.2 | 0.5/1.0 ml |

However, there are still problems in the aggregation of antibody products, which is intended to be controlled by the addition of small amphiphilic molecules. Fusion proteins, such as Etanercept, can generate a variety of degraded and aggregated products that can subsequently lead to reduced activity and even adverse effects such as immunogenicity. Therefore, there is still a need for a stable liquid formulation of the TNFR:Fc fusion protein, Etanercept. For this reason, there is a need to develop new liquid formulations that are capable of stably maintaining the activity of Etanercept for a long period and are effective in stabilizing current compositions of Etanercept.

These formulations must perform a variety of tasks. For example, the formulation must be physiologically acceptable and preferably provide an environment that guarantees the stability of the biopharmaceutical in a therapeutically effective concentration. In addition, it should allow for a satisfactory life span of the drug.

Therefore, the purpose of this invention is to provide pharmaceutical formulations for TNFR:Fc which may serve as an alternative to the previous known formulations.

### Brief Description of the Figures

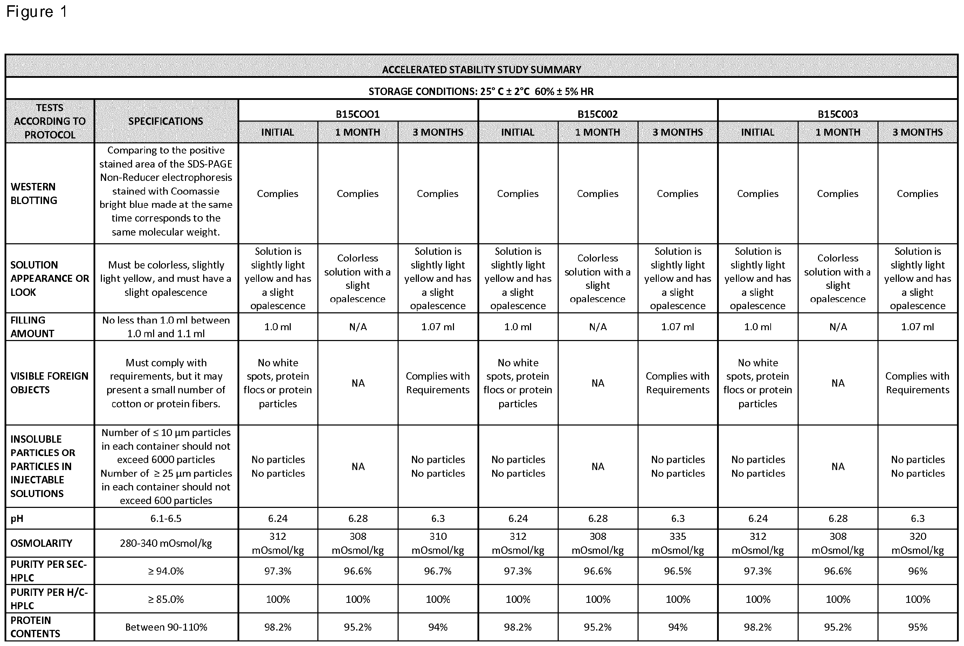

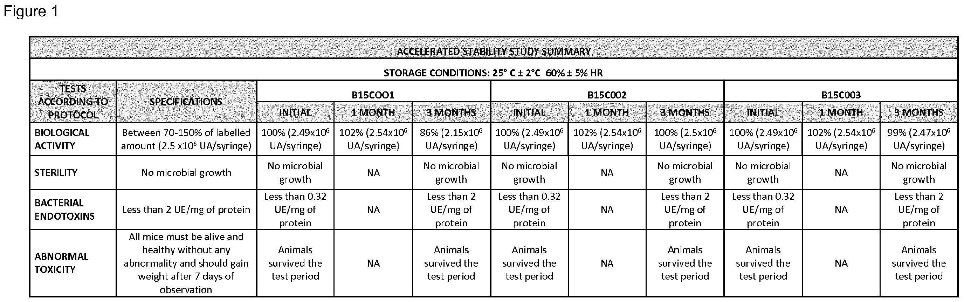

Figure 1 shows a summary table of the Accelerated Stability Study of the composition which is the subject of this invention.

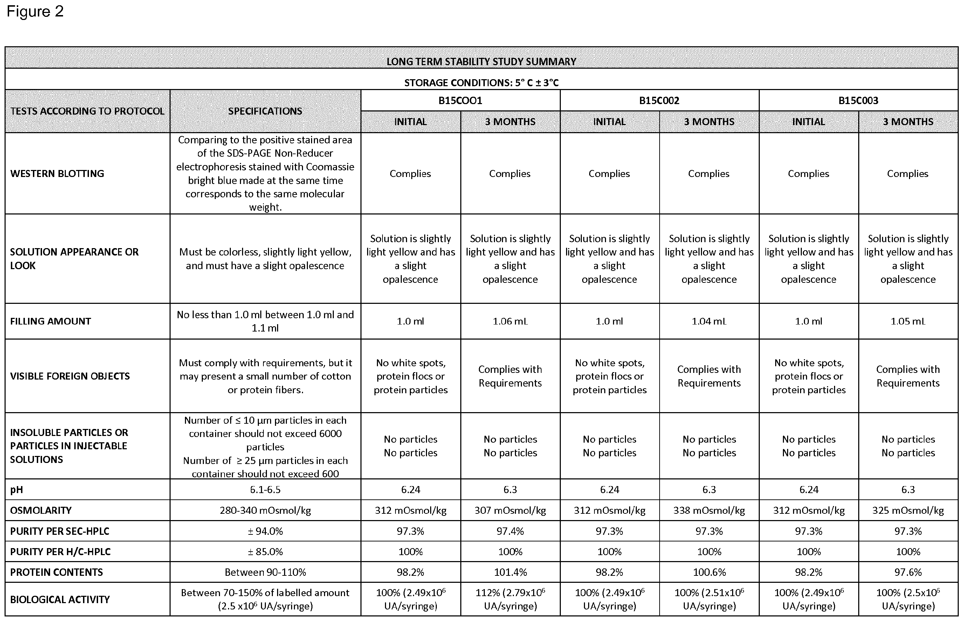

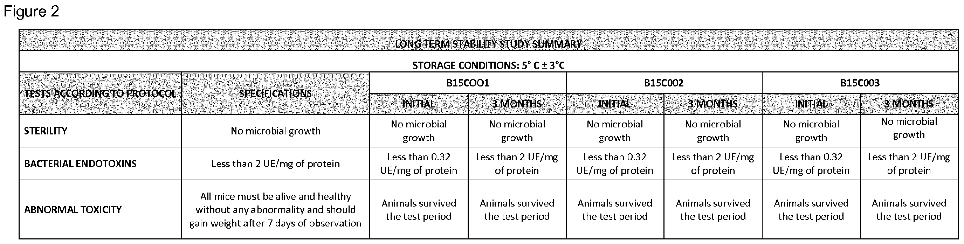

Figure 2 shows a summary table of the Long Term Stability Study of the composition which is the subject of this invention.

### Invention Description

The composition which comprises this invention expands the average life of Etanercept in aqueous solutions for subcutaneous administration. This extension of the average life is achieved by the addition of small amounts of an osmoregulator, which contributes significantly to the maintenance of homeostasis of the liquids. Osmoregulation is the active way to regulate the osmotic pressure of the internal environment and preserve the homeostasis of body fluids.

Consequently, the addition of a supplementary osmoregulator in the aqueous compositions of Etanercept contributes to the stability in the composition, avoiding chemical denaturation by decreasing the dissociation of the dimers that form the Etanercept molecule and avoiding the loss of tertiary structure, covalent/non-covalent aggregation and adhesion of monomers.

In general, previous compositions, as references in the documents from the Background section above, use a single stabilizing agent, which can also act as an osmoregulator and employ a single excipient, for example sucrose, to give tonicity to the composition. Even the composition of the Enebrel^{®} product, only uses a single stabilizing agent, I-arginine, and a single excipient, sucrose. The combination of a stabilizer and an osmoregulator is not considered as convenient. In fact, adding more than one stabilizer, or more than one excipient and even more to introduce several excipients or stabilization agents, is generally considered as undesirable under common best practices. Refer particularly to patent MX263192, where the use of a single stabilizing agent and a single excipient is emphasized in the description and claims. All this in order to avoid the introduction of compounds that affect the chemical and physical stability of Etanercept. Thus, the composition of this invention provides an improved stability composition by the addition of an additional osmoregulator, such as Mannitol.

The active ingredient of the composition is Etanercept, which is a recombinant fusion protein (rh TNFR:Fc) of Receptor II: IgG Fc of alpha-factor human tumor necrosis. This is a type of genetically engineered protein that is obtained from Chinese hamster ovary cells (CHO cells) as host cells and produced by recombinant DNA technology, which is a protein fused with PT5 receptor of TNF-alpha Type II and human Fc IgG1. Similar to the production process of genetic antibody engineering, the production process of the active principle for this product mainly includes 2 steps: cell culture and purification.

The dosage is undoubtedly one of the most relevant issues since the administration mode depends on the therapeutic effect desired. In this case, it refers to the treatment of inflammatory diseases with a tumor necrosis factor within its inflammation mechanism, which occurs mainly in autoimmune diseases such as rheumatoid arthritis and ankylosing spondylitis. Inevitably, the action site is in different parts of the human body, and, therefore, its bioavailability must be general. The conservation mode and temperature must also be considered since the protein is unstable under weather temperature conditions. Taking into account these factors, it was determined that the most suitable pharmaceutical form is a subcutaneous injectable solution, since it seeks to deposit the drug at the sub-dermal level, where it is absorbed and incorporated into the systemic circulation. Its advantages are: it requires a lower injectable volume, it is a route of rapid administration, it is less painful and the effect of the first step is avoided.

In addition, its pharmaceutical form, formulation, production process, container-closure system and microbiological conditions are appropriate for this specific purpose of administration.

The manufacturing process consists of the concentration of a bulk Etanercept 25 mg/ml solution. Through a tangential filtration or ultrafiltration process, the initial concentration of the solution is doubled, obtaining half of the initial solution by means of a particle size exclusion mechanism, in which only particles weighing less than 50 KDa can pass through the filter membrane obtaining a final concentration of 50 mg/ml in half of the initial solution volume. This process is of critical importance because of the protein concentration, which is higher, and thus allows dosing more protein in a smaller amount of solution; favoring the treatment to which the patient is subjected with the medication.

The composition of the final injectable solution contains Etanercept, an active principle used to treat various diseases of autoimmune origin. It acts by inhibiting the tumor necrosis factor. The tumor necrosis factor is a substance of the cytokines group that produces immune system cells and stimulates the acute phase of the inflammatory reaction.

Phosphate dibasic disodium heptahydrate is a substance that dissociates into four different ions that provide an acid or base to the solution depending on the concentration that is added in the solution, because this feature can be used to form a pH buffer solution.

Monosodium monobasic phosphate monohydrate also dissociates into four different ions that provide an acid or base depending on the amount present in the solution, so it is also used to form a pH buffer solution.

Arginine hydrochloride stabilizes the protein within the solution because it is an antioxidant agent, which protects the active degradation principle.

Sodium chloride regulates isotonicity in the solution by providing chlorine and sodium ions that increase the osmolarity of the solution in order to maintain the isotonicity of the final solution as close as possible to the patient's organic fluids.

Sucrose acts as an osmoprotector. In other words, it rises the osmotic pressure to maintain the turgor and the adequate hydric potential under stress conditions and therefore protects the integrity of the protein.

Mannitol is an osmoregulator. Osmoregulation is the active regulation the osmotic pressure of the internal medium by maintaining the homeostasis of the body fluids. In this sense, the mannitol acts as a solute gain in the solvent, which adds osmoles to the solution. This modifies the tonicity of the solution to favor the absorption of the solution at the application site by adding a favorable osmotic gradient to the surrounding medium.

Amounts are expressed as a component weight percentage (%) by over the total composition weight, or w/w.

The compositions included in this invention comprise 4%-5% of Etanercept; 0.45%-0.5% of arginine hydrochloride; 0.17%-0.21% of dibasic disodium phosphate heptahydrate; 0.2%-0.25% monosodium monobasic phosphate monohydrate; 0.5%-0.6% sodium chloride; 0.9% -1% sucrose; 0.18%-0.2% of mannitol and 92-93% of injectable grade purified water.

In particular, the compositions included in this invention comprise 50 mg of Etanercept; about 5.3 mg of arginine hydrochloride; 2.03 mg of dibasic disodium phosphate heptahydrate; about 2.4 mg of monosodium monobasic phosphate monohydrate; 5.84 mg of sodium chloride; 10 mg of sucrose; 2 mg of mannitol and 1 ml of injectable grade purified water.

The obtained composition is provided in syringes pre-filled with 1 ml of said composition. These pre-filled syringes are available for immediate application to the patient in need of Etanercept treatment. The syringes can be stored under controlled conditions for up to 24 months, so that Etanercept doses of immediate use will always be available at any time without having to proceed with the thawing of higher volume stocks.

The solution for pre-filling syringes with 50 mg/ml of Etanercept is prepared by the following procedure:
A 0.1 N sodium hydroxide solution is prepared, with which the tangential filtration equipment is washed and sanitized, the tangential filtration equipment is rinsed and washed with water for the manufacture of injectable syringes, thereby neutralizing the pH produced by the sodium hydroxide. The permeability test is performed during the last wash. The integrity of the cartridge membrane of the tangential filtration equipment is tested. Once the equipment is ready, the concentrated bulk solution of Etanercept, monobasic phosphate, dibasic phosphate, sucrose and sodium chloride is thawed.

Mannitol is added to the bulk thawed solution of Etanercept and mixed gently until mannitol has been fully incorporated into the final solution to avoid foaming. The above mixture is then passed through a 0.45 micron filter with the aid of a peristatic pump at a speed of 98-102 rpm. Next, the solution is passed through the tangential filtration equipment with 50 KDa membranes to obtain the concentrated solution.

The concentrated solution is passed through two 0.22 micrometer filters connected in tandem. The glass syringes are finally filled with the solution obtained in the previous step to a volume of 1-1.01 ml for the 50 mg/ml concentration and to a volume of 0.5 to 0.51 ml for the 25 mg/0.5 ml concentration.

### Stability Tests

The formulation of this invention maintains its physical and chemical identity and the integrity of the active ingredient, Etanercept, during storage in solution for long periods of time of up to 24 months. The stability can be measured by commonly used protein stability tests. Also, stability can be measured at a predetermined temperature for a predetermined period of time. For a quick test, the formulation can be stored at a high or higher temperature, for example 40° C for 2 weeks to a month or for a longer period, measuring its stability based on the length of this period.

Table 1 and 2 show the results of the accelerated stability studies and the long-term stability study where it can be seen that during the three-month stability test, the physical identity, chemistry and integrity of Etanercept contained in the composition included in this invention are preserved.

## Claims

1. The improved Etanercept composition stability, comprised of 4%-5% of Etanercept; 0.45%-0.5% of arginine hydrochloride; 0.17%-0.21% of dibasic disodium phosphate heptahydrate; 0.2%-0.25% monosodium monobasic phosphate monohydrate; 0.5%-0.6% sodium chloride; 0.9% -1% sucrose; 0.18%0.2% of mannitol and 92-93% of injectable grade purified water.

2. The composition from claim 1, comprised by 50 mg of Etanercept; 5.3 mg of arginine hydrochloride; 2.03 mg of dibasic disodium phosphate heptahydrate; 2.4 mg monosodium monobasic phosphate monohydrate; 5.84 mg of sodium chloride; 10 mg of sucrose; 2 mg of mannitol and 1 ml of purified water for injection.

3. The composition from claim 1, in the form of an injectable solution for pre-filled syringes.

4. Process for the preparation of the composition from claim 1, comprising of the following steps:
a. prepare a solution of Etanercept, monobasic phosphate, dibasic phosphate; sucrose and sodium chloride;
b. freeze the solution from step a);
c. prepare a 0.1 N sodium hydroxide solution,
d. wash and sanitize the tangential filtration equipment, rinse with water for the manufacture of injectable solution
e. perform a permeability test of the equipment filters;
f. test the integrity of the cartridge membrane of the tangential filtration equipment,
g. thaw the solution from step b);
h. add Mannitol to the thawed Etanercept solution
i. mix gently until mannitol is fully incorporated into the final solution
j. pass this mixture through a 0.45 micron filter with the aid of a peristatic pump at a speed of 98-102 rpm
k. pass the solution through the tangential filtration equipment with 50 KDa membrane
l. pass the solution through two 0.22 micrometer filters connected in tandem
m. fill the glass syringes with the solution obtained in the previous step to a volume of 1-1.01 ml

## Patentansprüche

1. Die verbesserte Stabilität der Etanercept-Zusammensetzung, bestehend aus 4 %-5 % Etanercept; 0,45 %-0,5 % Argininhydrochlorid; 0,17 %-0,21 % dibasisches Dinatriumphosphat Heptahydrat; 0,2 %-0,25 % monobasisches Mononatriumphosphat Monohydrat; 0,5 %-0,6 % Natriumchlorid; 0,9 %-1 % Saccharose; 0,18 %-0,2 % Mannitol und 92-93 % gereinigtes Wasser für Injektionszwecke.

2. Zusammensetzung nach Anspruch 1, bestehend aus 50 mg Etanercept; 5,3 mg Argininhydrochlorid; 2,03 mg dibasisches Dinatriumphosphat Heptahydrat; 2,4 mg monobasisches Mononatriumphosphat Monohydrat; 5,84 mg Natriumchlorid; 10 mg Saccharose; 2 mg Mannitol und 1 ml gereinigtes Wasser für Injektionszwecke.

3. Zusammensetzung nach Anspruch 1, in Form einer injizierbaren Lösung für Fertigspritzen.

4. Verfahren zur Herstellung der Zusammensetzung nach Anspruch 1, umfassend die folgenden Schritte:
a. Herstellung einer Lösung aus Etanercept, monobasischem Phosphat, dibasischem Phosphat, Saccharose und Natriumchlorid;
b. Einfrieren der Lösung aus Schritt a);
c. Herstellung einer 0,1 N Natriumhydroxidlösung;
d. Waschen und desinfizieren der tangentialen Filtrationsanlage, Spülung mit Wasser für die Herstellung der injizierbaren Lösung;
e. Durchführung einer Durchlässigkeitsprüfung der Gerätefilter;
f. Prüfung der Unversehrtheit der Patronenmembran der Tangentialfiltrationsanlage;
b. Auftauen der Lösung aus Schritt a);
h. Hinzufügung von Mannitol zur aufgetauten Etanercept-Lösung;
i. Vorsichtiges Vermischen, bis Mannitol vollständig in die fertige Lösung integriert ist;
j. Dieses Gemisch wird mit Hilfe einer Peristatikpumpe mit einer Drehzahl von 98-102 U/min durch einen 0,45-Mikron-Filter geleitet;
k. Die Lösung wird mit einer 50 KDa-Membran durch die tangentiale Filteranlage geleitet;
l. Die Lösung wird durch zwei miteinander verbundene 0,22-Mikrometer-Filter geleitet;
m. Die Glasspritzen werden mit der im vorherigen Schritt erhaltenen Lösung auf ein Volumen von 1-1,01 ml gefüllt.

## Revendications

1. L'amélioration de la stabilité de la composition d'Etanercept comprend 4%-5% d'Etanercept; 0,45%-0,5% de chlorhydrate d'arginine; 0,17%-0,21% de phosphate disodique dibasique heptahydraté; 0,2%-0,25% de phosphate monosodique monobasique monohydraté; 0,5%-0,6% de chlorure de sodium; 0,9%-1% de saccharose; 0,18%-0,2% de mannitol et 92-93% d'eau purifiée pour injection.

2. La composition selon la revendication 1 comprend 50 mg d'Etanercept; 5,3 mg de chlorhydrate d'arginine; 2,03 mg de phosphate disodique dibasique heptahydraté; 2,4 mg de phosphate monosodique monobasique monohydraté; 5,84 mg de chlorure de sodium; 10 mg de saccharose; 2 mg de mannitol et 1 ml d'eau purifiée pour injection.

3. La composition selon la revendication 1, sous forme de solution injectable pour seringues préremplies.

4. Processus de préparation de la composition à partir de la revendication 1, comprenant les étapes suivantes:
a. préparer une solution d'Etanercept, de phosphate monobasique, de phosphate dibasique, de saccharose et de chlorure de sodium;
b. congeler la solution à partir de l'étape a);
c. préparer une solution d'hydroxyde de sodium 0,1 N;
d. laver et désinfecter l'équipement de filtration tangentielle, rincer à l'eau pour la fabrication de la solution injectable;
e. effectuer un test de perméabilité des filtres de l'équipement;
f. tester l'intégrité de la membrane de la cartouche de l'équipement de filtration tangentielle;
b. décongeler la solution à partir de l'étape b);
h. ajouter mannitol à la solution d'Etanercept décongelée;
i. mélanger doucement jusqu'à ce que le mannitol soit complètement incorporé dans la solution finale;
j. faire passer ce mélange à travers un filtre de 0,45 microns à l'aide d'une pompe péristaltique à une vitesse de 98-102 tr/min;
k. faire passer la solution à travers l'équipement de filtration tangentielle avec une membrane de 50 kDa;
l. faire passer la solution à travers deux filtres de 0,22 micromètres connectés en tandem;
m. remplir les seringues en verre de la solution obtenue à l'étape précédente jusqu'à un volume de 1-1,01 ml.
